# EUROPEAN PATENT APPLICATION

(11) **EP 4 074 375 A1**
(43) Date of publication of application: **19.10.2022**
(21) Application number: 19955937.8
(22) Date of filing: 12.12.2019
(51) Int. Cl.: A61N 5/06

(54) **WOUND TREATMENT DEVICE AND MEANS OF USE**

(71) Applicant: Moreira Guimarães, Caio, São Paulo/SP (BR)
(72) Inventor: Moreira Guimarães, Caio, São Paulo/SP (BR)
(74) Representative: Ferreira Magno, Fernando Antonio
(86) International application number: PCT/BR2019/050535
(87) International publication number: WO 2021/113931

(57) **Abstract**

"DEVICE FOR WOUND TREATMENT AND MEANS OF USE", which deals with a new configuration applied to a device for wound treatment, for the medical field, where said device has the action of electromagnetic waves on parts of a patient's body, more specifically, a product for the improvement of the treatment of wounds of the body surface as those resulting from severe consequences and difficult healing, which can be used by diabetic or non-diabetic patients.

## Description

1. The present Patent of Invention deals with a new configuration applied to a device for treating wounds with the action of electromagnetic waves in parts of a patient's body, more specifically, a product for the improvement of the treatment of wounds of the body surface, such as those resulting from severe consequences and difficult healing, which can be used by diabetic or non-diabetic patients.

2. FUNDAMENTALS OF TECHNIQUE - Current devices that use electromagnetic wavelengths in wound treatments in diabetic patients or those requiring aid in wound healing are ineffective due to very time-consuming action, difficult access to asepsis, making it inadequate, to a smaller contact area and luminosity emitter range in the target body tissue, uncomfortable position for patient and professional who applies the treatment, difficulty in the use and price of the product, which often causes the patient to require one or several surgeries to remove tissue in degeneration or necrosis and recovery of the affected area to prevent or reduce the possibility of infectious processes installation, which raises treatment costs due to the need for hospitalization and new surgeries.

3. The worst-case scenario is that of diabetic patients, who have difficulty healing, especially in the region of the feet, known as "diabetic foot", since the ineffectiveness of auxiliary devices for the treatment of wounds leads to a high rate of amputations, as injured areas, even with small cuts or skin cracks, can easily infect and evolve to severe cases of gangrene and/or skin ulcer. The patient in these situations have the risk of amputation due to delay or even lack of healing and the inability of the devices to treat the most advanced cases infers in surgical intervention, often necessary despite the high mortality rate of 30% after 3 years of amputation of small regions.

4. Studies of the International Consensus on Diabetic Foot reveal that of 100% of patients with deep infections, 86% require surgery to obtain healing of the injured area. Amputations, at a lower level, are indicated to remove gangrene, but increase the risk of subsequent amputations. This situation was what motivated the studies and the present invention.

5. An important description of wound treatment time is in the Open Access Scientific Journal of the Public Library of Science, Plos One, in which a Paper was published on May 12, 2017 that reported that the early detection and referral for treatment are crucial for the healing of diabetic foot ulcers in a joint treatment by a specialist. In this study, the researchers proved that the average mean follow-up time of patients who participated in the study was 130 days, and from the beginning of treatment to healing, there was an average of 113 days.

6. In the researches carried out, we found in the Patent database of the PTO some patent applications and grantings related to this subject, among them:
7. PI 0406340-6, which describes a LED comprising equipment for therapeutic methods in order to promote acceleration of the healing of injured tissues.
8. BR 10 2012 022729 0, which describes a portable device for healing skin wounds. This device has a layer to absorb infrared radiation and transmits some of the radiation into the skin to promote healing through photobiomodulation.
9. PI 0705193-0, which describes a device for foot therapy through low voltage long infrared that comprises LED and is indicated to accelerate healing among other treatments.
10. WO 2018/039656, which describes a wound "band aid" that increases tissue healing using predetermined wavelength (of light).
11. WO 2009/008967, which describes a portable wearable device that uses cold laser to stimulate hair growth and skin treatment. The use of LEDs is cited.
12. CN 204232399, which describes insoles and shoes for personal care and heat preservation using LED to stay protected from the winter cold.
13. CN 204582326, which describes smart footwear that comprises photocurrent generator including a lighting circuit with a plurality of LED source.
14. CN 206197189, which describes shoes for phototherapy with LED source, LED circuit for radiation treatment.
15. WO 2017/010685, which describes a medical wound healing accelerator and osteomyelitis treatment device. This accelerator includes a plurality of light source units to emit into light skin providing a beneficial effect for wound.
16. RU 2526475, which describes a method of physiotherapeutic treatment of patients with diabetic neuropathy of low extremities such as the feet. The use of infrared radiation is cited.
17. US 6,810,288, which describes a wound healing device comprising ultraviolet light generator and a photoativable material, indicated for use in the feet.
18. US 20100324455, which describes devices for the care of foot injuries including foot ulcers. It is predicted use of light source and can be LED.
19. US 20150196771, which describes devices for treating degenerative joint diseases using magnetic fields using specific wavelength to reduce pain and inflammation and improve tissue healing and regeneration.

20. There are scientific publications on the subject, in which we also find information that currently, professionals such as doctors physicians, physiotherapists, dentists, among others, provide adjuvant therapeutic resources that describe wound healing processes using lengths of electromagnetic waves through emitters such as LED, laser or others.

21. However, several drawbacks are encountered when analyzing these anteriority documents: most documents deal with the LED itself or another emitter for the application in wounds with the purpose of healing, however, there is no efficient, modern, light, hygienic, portable device, easy access for asepsis and practicality in use, and it was thought to overcome all these obstacles that the inventor developed a device, easily applicable to feet, with removable partitions that can be used also on other parts of the body. Said apparatus, comprising sources of emission of electromagnetic waves throughout its length to be applied for acceleration of wound healing, inflammatory control and bactericidal and bacteriostatic effect with openings that allow the perfect conditioning and maintenance of the foot position during its treatment, as well as ventilation, correct distance between wave emitters and the skin, easy to use and to make the asepsis.

22. There are no documents suggesting a detachable device with configuration conducive to the treatment and arrangement of plates of electromagnetic wave emission sources as demonstrated in the figures attached to this document.

23. There are, in the state of the art, specific disadvantages because no device was found that is constituted in such a way as to facilitate and make the application of the treatment more effective, both from the point of view of professionals, when we talk about the effectiveness, asepsis of the device, fitting, agility and practicality of application, as well as from the point of view of patients, when we talk about better position, hygiene, effective proximity of electromagnetic wave emitters to the target surface, comfort and satisfactory result.

24. Lack of hygiene is an important point to be taken into account, since wounds necessarily need an aseptic and ventilated environment and material to heal, with a certain temperature control for them to be treated. The conditions of asepsis, ventilation control and temperature aim to reduce the possibility of aggravation of the lesion, as well as the intensification of the infectious process, as is the case of osteomyelitis.

25. Taking into account everything that has been described, as well as the disadvantages presented in the products of the state of the art, we have: devices with configuration ready to be used in only one part of the body, without adaptation to the feet; electromagnetic wave emitters in specific parts of the device that do not meet the emission of waves to specific parts of the body and in positions that do not leave the delivery of electromagnetic emission to the target surface throughout the treatment; lack of ventilation of the appliances impairing healing; non-dismountable devices with difficult access for asepsis; discomfort of the patient, as he/her should remain in the same position, sometimes uncomfortable, until treatment is finished; lack of a display designed to facilitate the operation of the device and lack of a weight on the base of the device that causes an effect like the so-called "fool-up" that will give ergonomics and free movement to the foot, when inserted into the device, even in a relaxing position.

26. OBJECTIVES OF THE INVENT - The present aims to provide a device designed for the treatment and improvement of skin ulcers in which, according to all application tests, it was possible to reduce to at least ¹/₄ the healing time, as pointed out in the studies conducted, in which advanced-stage skin ulcers and in diabetic patients are healed between 8 and 15 weeks, even without the use of ointments, and may also serve as an auxiliary device to other treatments, since it potentiates the healing effects.

27. Treatment with this device has a lower cost due to the drastic reduction of the healing time of the lesion, the decrease in the number of hospitalizations and surgical procedures, as well as the possibility of reducing the use of medication by patients.

28. Developed with the objective of providing easy use, both by the medical team and by the patient himself, since the products of the state of the art do not present practical management functions, they are not light (in terms of weight) products and with no adequate configuration, and the patient suffers from discomfort and lack of asepsis in the use and moves during treatment, touching the skin on the wave emitters, and, therefore, to solve these problems it is hereby presented a product that can be dismountable, and its parts, when disassembled, may also emit electromagnetic waves with therapeutic effects in other parts of the body than not only the feet of the patient. Said product, after use, can easily be reassembled, cleaned and have parts that eventually touch the skin exchanged.

29. Taking into account all informed advantages, we list the improvements achieved in the device: dismountable and retableable device, which can radiate electromagnetic waves, in different lengths, with beneficial therapeutic effects in any part of the body, but especially adaptable to the region of the feet; electromagnetic waves throughout the internal extension of the device, which however reach any portion of the body, since its parts can be disassembled and, after analysis, the healthy professional chooses the configuration that best affects the wound of the patient's body; ventilated device that does not hold the patient's foot, but leaves it comfortably in correct condition and position for non-complication of ulcers; dismountable and retableable, which facilitates asepsis to the fullest extent; welcomes and fits the patient's foot, and its lower part receives a ballast weight with a "Dot-bobo" effect, in which the low center of gravity near the rounded base of the object leaves it stable because it concentrates the weight at the base and for this reason, even if the patient is relaxed or asleep, the foot will tend to the vertical position, properly receiving the waves and ventilation. With the heavier base, the tendency is the vertical position of the foot, without touching the skin directly on the electromagnetic wave emitters and maintaining space for ventilation, which increases the potentiation of the treatment.

30. DESCRIPTION OF FIGURES - For a better and adequate understanding of the Patent of Invention, it is now described below with the help of the attached figures, where:
31. Figure 1 illustrates a layout of the parts of the device in an exploded view;
32. Figure 2 shows the front of the body of the two-sided device, without the lower portion;

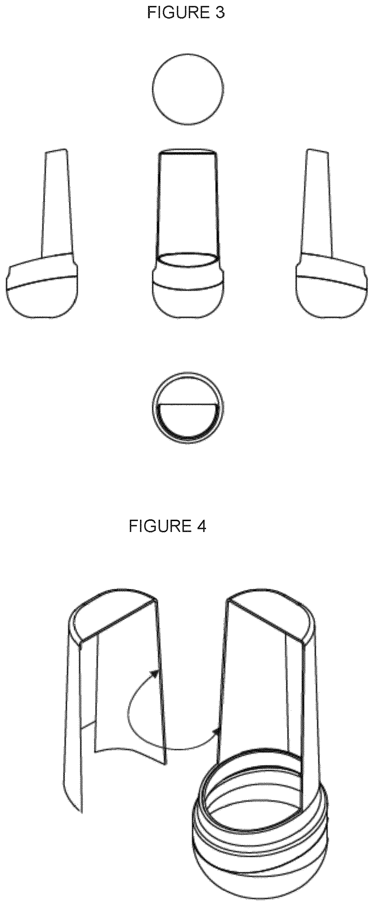

33. Figure 3 illustrates another view of the device with the lower portion, without the front, in an exploded view;
34. Figure 4 shows the front separated from the back portion with the snap-on motion, illustrating that the walls of the device can join together;

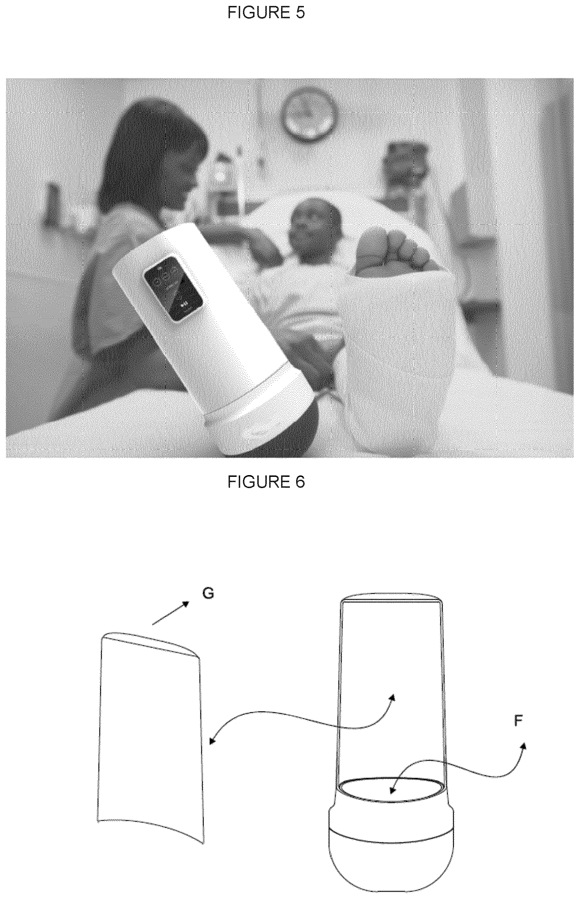

35. Figure 5 shows one of a patient's feet inserted in a version of the device;
36. Figure 6 illustrates a transparent piece of plastic or other disposable transparent material or not to be fitted inside the device;

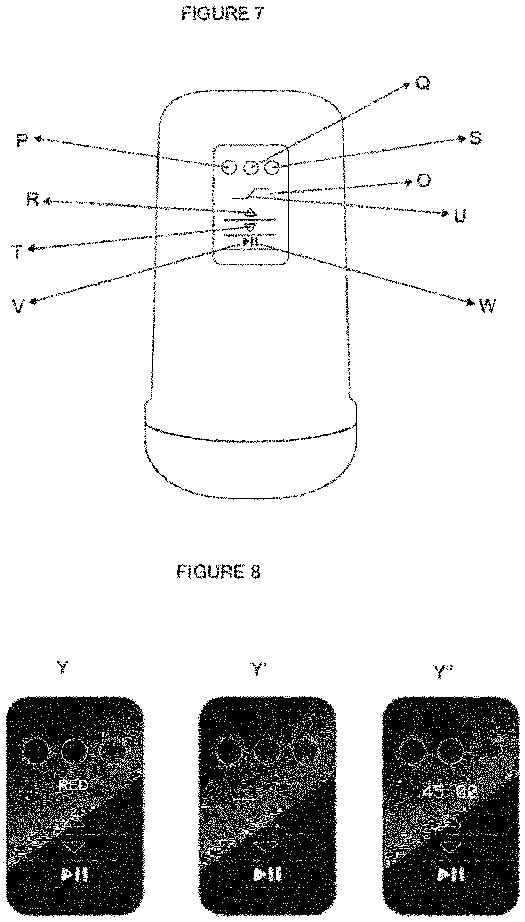

37. Figure 7 illustrates the rear view of the device, with a diagram of the device's drive buttons;
38. Figure 8 shows the three types of drive buttons that can be seen when programming: color, mode and time;

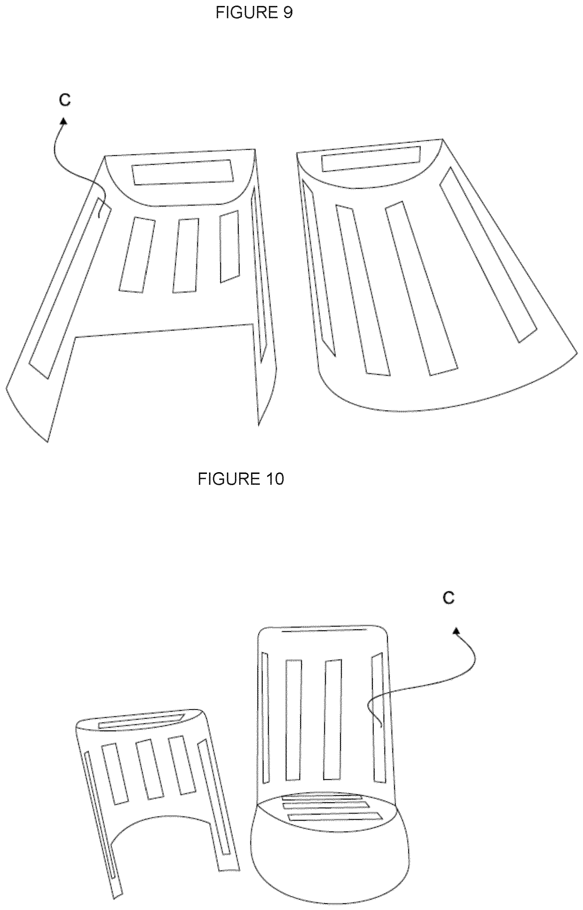

39. Figures 9 and 10 illustrate the internal part of the device where many bands of wave emitters can be observed throughout their length, showing possibilities of arrangement of some electromagnetic wave emitters inside the device;

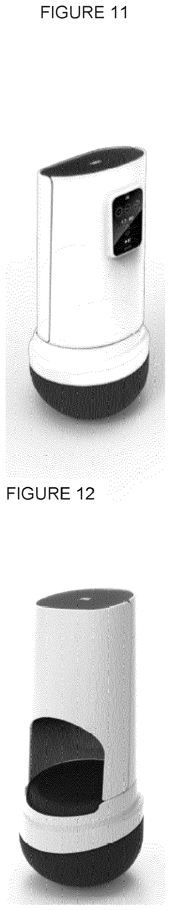

40. Figures 11 and 12 show new views of the device;

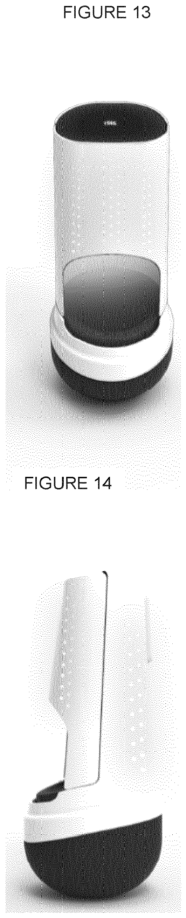

41. Figures 13 and 14 show views of the device with waves in blue;

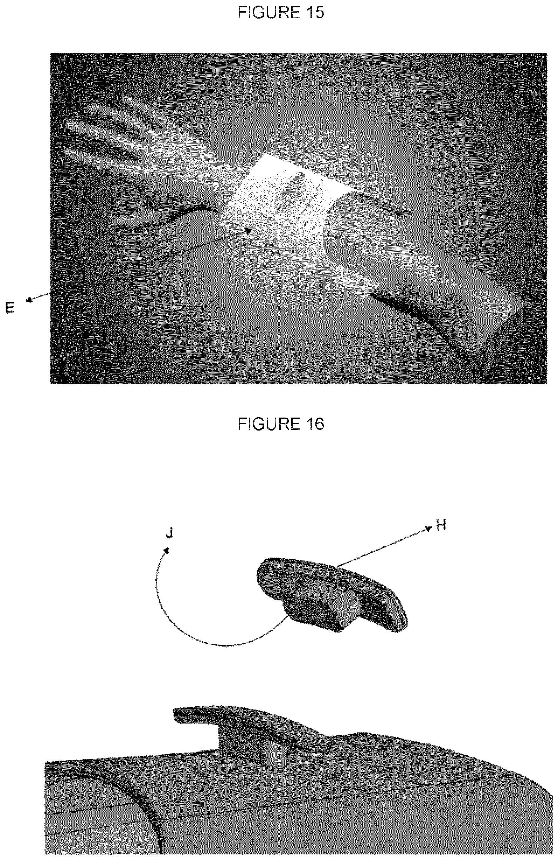

42. Figure 15 shows wounds of patient M. P.S. at the beginning of treatment and every 10 sessions, corresponding to 70 days of treatment with the respective graph of the statistics and the percentage of ulcer reduction;
43. Figure 16 shows the clinical-evolutionary aspects of diabetic toe ulcers of patient C.J.G. O. at the beginning of treatment and every 10 sessions, corresponding to 8 weeks of treatment with the respective graph of the statistics and percentage of ulcer reduction.

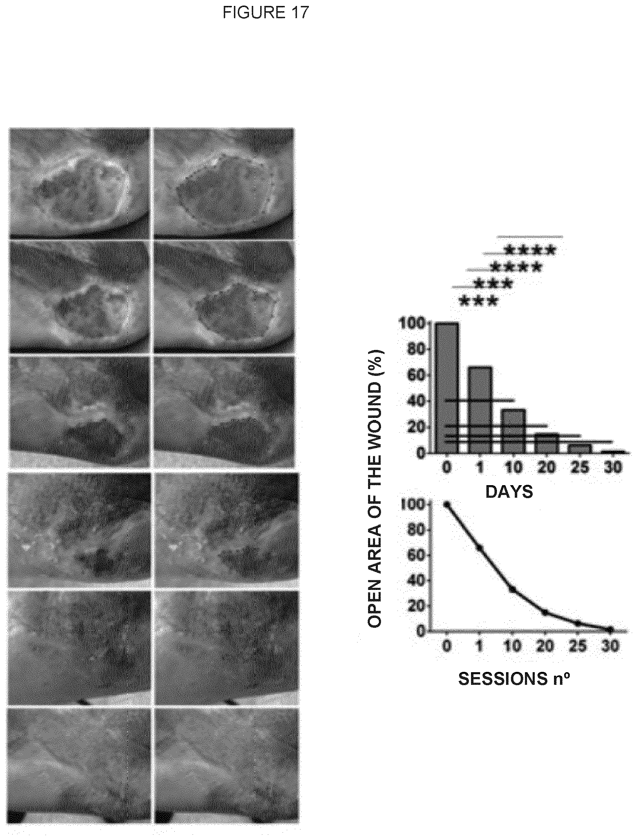

44. Figure 17 shows clinical-evolutionary aspects of diabetic foot stump ulcers of patient H.M.S. at the beginning of treatment and every 10 sessions, corresponding to 11 weeks of treatment with the respective graphic of the statistics and the percentage of ulcer reduction; and

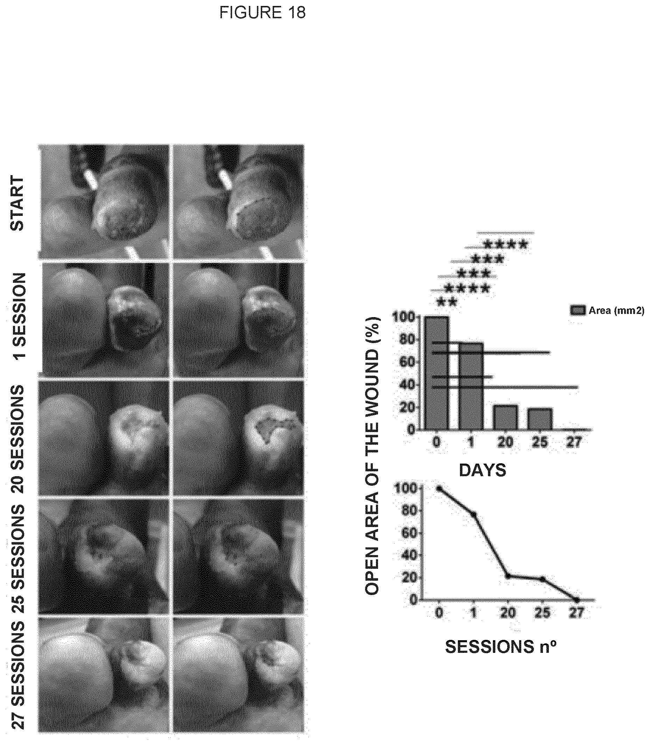

45. Figure 18 shows clinical-evolutionary aspects of diabetic foot sole ulcers of patient K.C. at the beginning of treatment and every 10 sessions, corresponding to 6 weeks of treatment with respective graph of the statistics and percentage of ulcer reduction.

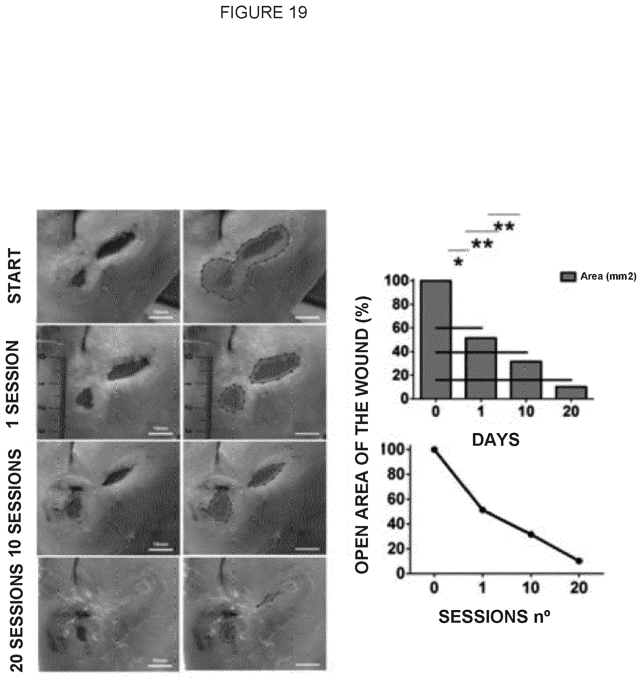

46. Figure 19 shows clinical-evolutionary aspects of diabetic foot stump ulcers of patient H.M. S. at the beginning of treatment and every 10 sessions, corresponding to 11 weeks of treatment with the respective graph of the statistics and the percentage of ulcer reduction; and

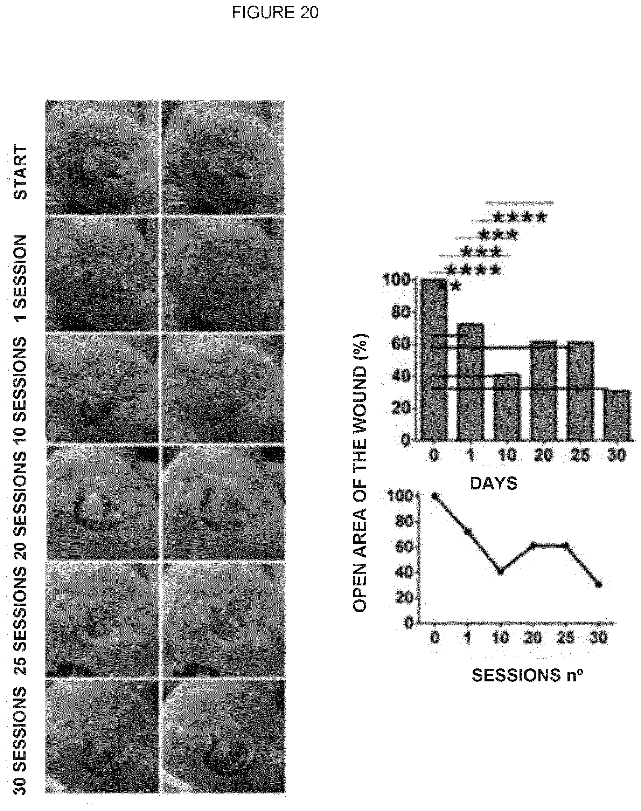

47. Figure 20 shows clinical-evolutionary aspects of diabetic foot sole ulcers of patient K.C. at the beginning of treatment and every 10 sessions, corresponding to 6 weeks of treatment with respective graph of the statistics and percentage of ulcer reduction.

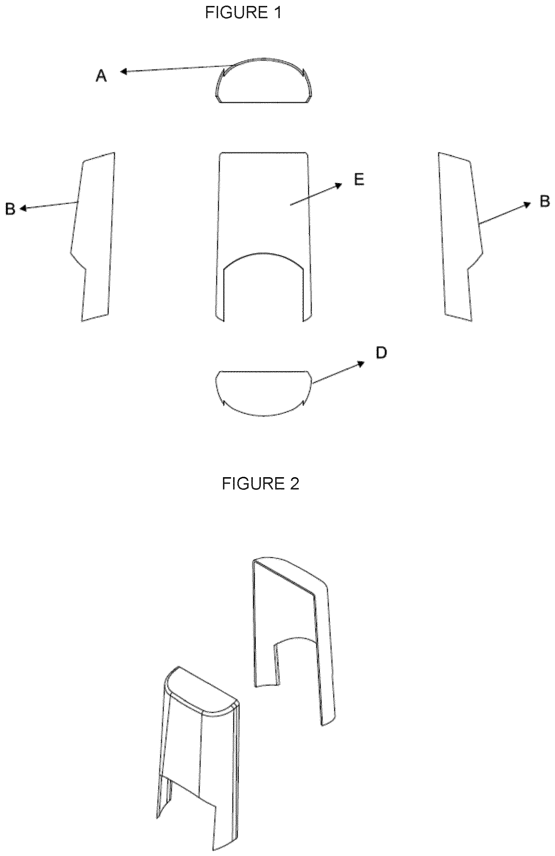

48. DETAILED DESCRIPTION - According to figures 1 to 1 4, the Invention comprises a device (13 and 14), for wound treatment (15 to 18), through electromagnetic wave emitters (C), where said device (13 and 14) can be configured through a panel (7) arranged in its posterior part (E); said panel (7) receives color buttons - wave length (Y), function (Y') and time (Y"), adjustable, for application (V) of these electromagnetic waves (Y), programmed by irradiation mode (Q) and time (S) in the manner desired by the professional, in order to heal the wounds.

49. The device (13 and 14) has in all its parts (A, B, D, E and G) click and/or magnet fittings and where the front (D), posterior (E) and internal transparent joints (G) can be detachable, as well as these elements (A, B, E and G) have interconnection between each other and with the lower portion (D) to be able to function. These elements (A, B, D and E) receive electromagnetic wave emitters internally (9 and 10) and can be used separately (B, D and E), applied to parts of the body other than the feet, with the same efficiency of the device (13 and 14) with all parts joined for application on the feet.

50. The configuration buttons are installed in the posterior portion of the device (E), through them you can determine the operating pattern of the frequency (Y'), color-wavelength (Y) and duration time (Y") of the waves of the emitters (C), of any of the parts (A, B, D and E), for the treatment of the patient.

51. Said device (13 and 14), can receive handles (H), which are attached to the parts (A, B, D, E and G), through magnets (J) when the device (13 and 14) is disassembled.

52. For operation, after programming the appliance, the most appropriate position is defined for the patient and in the case of the foot, for example, the patient's foot can be inserted into the device (5), where he/she may lie or sit down receiving the irradiation of electromagnetic waves (Y), comfortably and without accidentally removing the foot from the device, even if the body moves, as the base of the device receives an appropriate weight that helps to keep the foot in the correct position, even at the time of extreme relaxation of the patient.

53. The rear part of the device (B), always fixed, receives a polycarbonate plate with programming for the electronic component (7), where the programming begins with the color button (P), and each time it is pressed, shows on panel (O) the selection of the wavelength (Y) blue, red or blue + red; then the mode button (Q) where the continuous irradiation images appear, ramp or pulsed; at that point, if you want to set the time, the time button (S) is pressed and the treatment time is set, which will start when the play (V) button is pressed. Then a countdown will start, and when time runs out, the equipment shuts down. If user do not want to set the time, just presses play (V) and the treatment will start with a progressive count on the display. The equipment will only be switched off if the play button (V) is pressed again.

54. With regard to waves (Y') one can choose: Simultaneous, where all colors - wavelength - will be part of the treatment; ramp, where the wavelength power increases gradually - as scheduled; pulsed, where the wavelength is pulsating, increasing and decreasing or continuous, where the wavelength is frequent, without change.

55. The time button (S) when triggered once has the possibility to change the digits (Y"), where the button with the up arrow (R) increases said digit and with the down arrow (T) it decreases the programming time, progressively or backwardly. If a time schedule is not required, color and mode are triggered, and the time count will be progressive.

56. The plates with the electromagnetic wave circuit (C) have the arrangement with the wavelength of blue color throughout the contour of the device and the wavelength of red color more in the middle of the blue. This provision has the advantage that because blue light is less intense it needs to occupy a larger area, which makes the device (12 and 13) a capsule completely filled with electromagnetic emitters (C). Wavelengths are in blue and red colors can be used individually or simultaneously. If one needs to stop the emission, the pause button (W) can be triggered. To eliminate the disadvantage of hygienic conditions presented in other products, said device (13 and 14), disposable plastic socks can be used since electromagnetic waves will continue to be the same effectiveness, and may also have transparent parts(G) plastic or other disposable or washable material that are attached inside the parts (B, D and E) to be embedded inside the device (13 and 14) to prevent the patient's skin from contacting the walls of the device directly, and said transparent parts (G) also have simple snap and/or magnet fittings and can receive asepsis for new use or even be discarded. In the case of transparent plastic parts (G), the effectiveness of electromagnetic wave projection was proven in tests performed. The inner part (F) of the base (D) of the device (13 and 14) has acrylic disc shape or similar material, with conditions of transparency with a slight depression in the center, in the background, with the advantage that such concavity receives the heel anatomically. Said base (D), externally, has rubberized bottom to create resistance near the ground or support site, and internally, a ballast weight reinforces the effect "come and go".

57. The joining of the click and/or magnet parts will preferably be performed by male and female fittings, where these fittings will preferably receive magnets that will help in locking them.

58. The conduction of energy, when the parts are disunited can be carried out through cables with male and female plugs.

59. There may be an internal cooler for ventilation of the appliance, helping to ensure that the foot is always airy.

60. There is no direct contact with electromagnetic wave emitters (C) as they are covered by an acrylic cover, or similar material with transparency conditions. This cover can be removed when maintaining the transmitters.

61. Based on comparative checks, the results achieved with this device exceed expectations when related to technologies known for this same purpose.

## Claims

1. **"DEVICE FOR WOUND TREATMENT AND MEANS OF USE",** which presents a new configuration, for wound treatment, **characterized by** being designed for the treatment and improvement of skin ulcers, mountable and dismountable, which emit electromagnetic waves with therapeutic effects; treating wounds (15 to 18), through electromagnetic wave emitters (C), where said device (13 and 14) is configured through a panel (7) arranged in its posterior part (E); said panel (7) receives color-wave length buttons (Y), function (Y') and time (Y"), adjustable, for application (V) of these electromagnetic waves (Y), irradiation mode (Q) and time (S).

2. **"DEVICE FOR WOUND TREATMENT AND MEANS OF USE",** according to claim 1, said device being **characterized by** radiating electromagnetic waves in different lengths from the entire internal extension of the device, reaching any part of the body.

3. **"DEVICE FOR WOUND TREATMENT AND MEANS OF USE",** according to claim 1, said device **characterized by** being able to receive asepsis throughout its extension, inclusively when demounted.

4. **"DEVICE FOR WOUND TREATMENT AND MEANS OF USE",** according to claim 1, said apparatus being **characterized by** receiving the foot or other parts of the patient, and its lower portion receiving a ballast weight with a "Dot-fool" effect, in which the low center of gravity near the rounded base of the object leaves it stable, keeping yet space for ventilation between the human body part and the apparatus.

5. **"DEVICE FOR WOUND TREATMENT AND MEANS OF USE"** according to claim 1, **characterized by** the device (13 and 14), having in its parts (A, B, D, E and G) fittings where the front (D), posterior (E) and internal transparent joints (G) can be detachable; these elements (A, B, E and G) having interconnection between themselves and with the lower portion (D) and the elements (A, B, D and E) receiving electromagnetic wave emitters internally (9 and 10) and can be used separately (B, D and E), applied in parts of the body, other than feet.

6. **"DEVICE FOR WOUND TREATMENT AND MEANS OF USE",** according to claim 1, **characterized by** the device having configuration buttons installed in the posterior part (E) and through them allowing the determination of the operating pattern of the frequency (Y'), color - wavelength (Y) and duration time (Y") of the waves of the emitters (C), of any of the parties (A, B, D and E).

7. **"DEVICE FOR WOUND TREATMENT AND MEANS OF USE",** according to claim 1 **characterized by** the device (13 and 14), being able to receive handles (H), which are attached to the pieces (A, B, D, E and G), through magnets (J) when the device (13 and 14) is dismantled.

8. **"DEVICE FOR WOUND TREATMENT AND MEANS OF USE",** according to claim 1, **characterized by** the fact that its operation takes place through the programming of the apparatus.

9. **"DEVICE FOR WOUND TREATMENT AND MEANS OF USE",** according to claim 1, **characterized by** the back of the device (B) being fixed and receiving a polycarbonate plate with the programming for the electronic component (7), where the programming is started by the colored button (P), where, when it is pressed, the panel (O) shows the selection of the wavelength (Y), being blue, red or blue + red; then the mode button (Q) where when pressed shows continuous, ramp or pulsed irradiation images; at that time, if user want to set the time, the time button (S) is pressed and the treatment time is set, which will start when the play button (V) is pressed, when a countdown will start, and when the time runs out, the equipment turns off.

10. **"DEVICE FOR WOUND TREATMENT AND MEANS OF USE",** according to claim 1, **characterized by** the programming being initiated via the play button (V) with a progressive count on the display and by the fact that the equipment will only be switched off if the play button (V) is pressed again.

11. **"DEVICE FOR WOUND TREATMENT AND MEANS OF USE",** according to claim 1, **characterized by** waves (Y') being: Simultaneous, where all colors (wavelength) will be on for the treatment; ramp, where the wave length power increases gradually (as scheduled); pulsed, where the wavelength is pulsating, increasing and decreasing or continuous, where the wavelength is frequent, without change.

12. **"DEVICE FOR WOUND TREATMENT AND MEANS OF USE",** according to claim 1, **characterized by** the fact that the time button (S), when triggered once, has the possibility to change the digits (Y"), where the button with the up arrow (R) increases said digit and with the arrow down (T) it decreases the programming time, progressively or regressively and, if a time schedule is not requested, color and mode are triggered and the time count will be progressive.

13. **"DEVICE FOR WOUND TREATMENT AND MEANS OF USE",** according to claim 1, **characterized by** the plates with the electromagnetic wave circuit (C) having the arrangement with the wavelength of blue and red color throughout the contour of the device, the device being a capsule filled with electromagnetic emitters (C) where the blue and red wavelengths can be in used individually or simultaneously.

14. **"DEVICE FOR WOUND TREATMENT AND MEANS OF USE",** according to claim 1, **characterized by** the device having a pause button (W).

15. **"DEVICE FOR WOUND TREATMENT AND MEANS OF USE"** according to claim 1, **characterized by** the device (13 and 14), being able to be used with disposable plastic socks or transparent parts (G) plastic or other disposable or washable material attached to the inside of the parts (B, D and E) to be fitted to the inside of the device (13 and 14), and said transparent parts (G) also having fittings.

16. **"DEVICE FOR WOUND TREATMENT AND MEANS OF USE",** according to claim 1, **characterized by** the internal part (F) of the base (D) of the device (13 and 14) having disc format with transparency with a depression in its center, in the background and base (D) externally, having a rubberized base.

17. **"DEVICE FOR WOUND TREATMENT AND MEANS OF USE",** according to claim 1, **characterized by** the junction of the parts being carried out by male and female fittings, where these fittings receive magnets.

18. **"DEVICE FOR WOUND TREATMENT AND MEANS OF USE",** According to claim 1, **characterized by** the conduction of energy, when the parts are disunited, being carried out through cables with male and female plugs.

19. **"DEVICE FOR WOUND TREATMENT AND MEANS OF USE",** according to claim 1, **characterized by** the device structure permitting to receive an internal cooler.

20. **"DEVICE FOR WOUND TREATMENT AND MEANS OF USE",** according to claim 1, **characterized by** electromagnetic wave emitting links (C) being covered by a transparent and removable cover.

## Amended claims

### Amended claims under Art. 19.1 PCT

1. **"DEVICE FOR WOUND TREATMENT AND MEANS OF USE",** which treats wounds through electromagnetic wave emitters that presents a new configuration, characterized to be demountable and remedial treating wounds (15 to 18), through electromagnetic wave emitters (C) distributed throughout its length, configured through a panel (7) arranged in its posterior part (E); said panel (7) receives buttons of color
- wavelength (Y), function (Y') and time (Y), adjustable, for application (V) of these electromagnetic waves (Y), programmed by irradiation mode (Q) and time (S); that welcomes the foot or other parts of the patient, and its lower portion receives a ballast weight with a "Dot-fool" effect, maintaining ventilation space between the body part and the apparatus; having in all its parts (A, B, D, E and G) fittings where the front (D), posterior (E) and internal transparent joints (G) can be detachable and the elements (A, B, E and G) have interconnection between each other and with the lower portion (D) and receive electromagnetic wave emitters internally (9 and 10) that can be used separately (B, D and E), applied in other parts of the body, other than the feet; with configuration buttons installed in the later portion (E) that can determine the operating pattern of the frequency (Y'), color - wavelength (Y) and duration time (Y") of the waves of the emitters (C), of any of the parts (A, B, D and E); with the rear part of the device (B) fixed and containing a polycarbonate plate with programming for the electronic component (7), where the programming begins with the color button (P), which each time it is pressed, shows on panel (O) the selection of the wavelength (Y) blue, red or blue + red, then the button (Q) where pressing the continuous irradiation images appear, ramp or pulsed; with time button (S), play button (V), countdown and progressive, automatic and manual shutdown; with simultaneous (Y') waves, ramp and pulsed; Time button (S) when triggered once has the possibility to change the digits (Y"), where the button with the up arrow (R) increases said digit and with the down arrow (T) it decreases the programming time, with electromagnetic wave circuit boards (C) with the wavelength of blue and red color across the contour of the device, the device being a capsule filled with electromagnetic emitters (C) where blue and red wavelengths can be used individually or simultaneously; pause button (W); can be used with disposable plastic socks or transparent parts (G) plastic or other disposable or washable material that are attached to the inside of the parts (B, D and E) to be fitted inside the device (13 and 14), and said transparent parts (G) also have fittings; internal (F) part of the base (D) of the device (13 and 14) with disc format with transparency with a depression in the center, at the bottom and said base (D) and externally, have rubberized bottom; the joining of the parts carried out by male and female fittings containing magnets; energy conduction, when the parts are disunited be carried out through cables with male and female plugs; with internal cooler and electromagnetic wave emitters (C) covered by a transparent, removable cover.
